# EUROPÄISCHE PATENTANMELDUNG

(11) **EP 1 792 924 A1**
(43) Veröffentlichungstag der Anmeldung: **06.06.2007**
(21) Anmeldenummer: 05026266.6
(22) Anmeldetag: 01.12.2005
(51) Int. Cl.: C08F 220/38, A61L 27/14

(54) **Schwefelhaltige Monomere mit hohem Brechungsindex, deren Homo- und Copolymerisate sowie deren Verwendung in optischen Elementen**

(71) Anmelder: Coronis GmbH, 82152 Martinsried (DE)
(72) Erfinder: Storsberg, Joachim Dr., 55286 Wörstadt (DE); Laschewsky, André Prof. Dr., 14469 Potsdam (DE); Müller-Lierheim, Wolfgang Dr., 81243 München (DE)
(74) Vertreter: Eisenführ, Speiser & Partner

(57) **Zusammenfassung**

Es werden Monomere der Formel PG-Z-VG-(S-Aryl)ₙ, worin n eine ganze Zahl von 2 bis 5 ist, PG eine polymerisierbare Gruppe ist und VG eine Verknüpfungsgruppe ist und Z O, NH oder eine Bindung bedeutet, wobei die polymerisierbare Gruppe mindestens eine polymerisierbare Doppelbindung aufweist und wobei die Verknüpfungsgruppe eine geradkettige, verzweigte oder cyclische Alkylgruppe mit 1 bis 12 Kohlenstoffatomen oder eine Arylgruppe mit 6 bis 18 Kohlenstoffatomen ist, die gegebenenfalls bis zu drei Heteroatome ausgewählt aus O, S und N enthalten kann, beschrieben, sowie daraus hergestellte Polymere und deren Verwendung.

## Beschreibung

Die vorliegende Erfindung betrifft schwefelhaltige Monomere mit hohem Brechungsindex, deren Homo- und Copolymerisate sowie deren Verwendung in optischen Elementen.

Für optische Anwendungen sind Polymere wünschenswert, die einen hohen Brechungsindex und eine hohe Lichtdurchlässigkeit aufweisen. Im Stand der Technik sind Polymermaterialien verfügbar, die z.B. zur Herstellung von Intraokularlinsen Anwendung finden, die einen Brechungsindex von maximal 1,56, gemessen gegen Luft, haben. Eine Maßnahme, um den Brechungsindex weiter zu erhöhen, wird in WO 03/001278 A2 beschrieben. Hierzu werden anorganische Nanopartikel verwendet, die im sichtbaren Wellenlängenbereich des Lichtes transparenten hochbrechenden Polymermaterialien zugefügt werden.

Es war nun Aufgabe der vorliegenden Erfindung, Polymere bereitzustellen, deren Brechungsindex gegenüber dem von bekannten Materialien erhöht ist und Monomere bereitzustellen, die zu im sichtbaren Wellenlängenbereich des Lichtes transparenten hochbrechenden Polymermaterialien polymerisiert werden können.

Diese Aufgabe wird gelöst durch Monomere, wie sie in Anspruch 1 definiert werden, und daraus erzeugte Polymermaterialien.

Es wurde gefunden, dass die Monomeren der Formel (I), wenn sie zu Polymeren oder Copolymeren verarbeitet werden, Materialien mit einem hohen Brechungsindex, der weit über 1,56 liegt, liefern. Der stark verbesserte Brechungsindex wird durch mindestens zwei über Schwefel gebundene Arylgruppen in der Polymerseitenkette erreicht. Darüber hinaus können mit den erfindungsgemäßen Monomeren Polymere hergestellt werden, die sich durch hohe optische Transparenz auszeichnen, wie sie für optische Anwendungen erforderlich ist. Ein weiterer Vorteil der neuen Monomere bzw. der daraus hergestellten Polymerisate ist die hohe optische Absorption im UV-Bereich.

Die erfindungsgemäßen Monomere können mit Standardverfahren oder zu Standardverfahren analogen Verfahren hergestellt werden, wie unten näher erläutert.

Die erfindungsgemäßen Monomere können in an sich bekannter Weise zu Polymeren umgesetzt werden, wobei sowohl Homopolymere als auch Copolymere erhältlich sind, die über die für optische Anwendungen geforderten Eigenschaften verfügen. Unter "Copolymer" soll im Zusammenhang mit der vorliegenden Erfindung jedes Produkt verstanden werden, das aus zwei oder mehr verschiedenen Monomeren polymerisiert wurde und das neben den erfindungsgemäßen Monomeren weitere Monomere enthalten kann, wobei unter dem Begriff "Copolymer" sowohl solche Polymere zu verstehen sind, die aus zwei oder mehr unterschiedlichen erfindungsgemäßen Monomeren aufgebaut sind, als auch Polymere, die aus mindestens einem erfindungsgemäßen Monomer und weiteren bekannten Monomeren aufgebaut sind. Monomere, die Eigenschaften in gewünschter Art und Weise verändern, können zugefügt werden. Solche Monomere sind dem Fachmann bekannt und die jeweils geeigneten Monomere und Anteile können ggf. durch Routineversuche leicht aufgefunden werden. Da die erfindungsgemäßen Monomere einen sehr hohen Brechungsindex aufweisen, ist es möglich, zur Verbesserung von anderen Eigenschaften der Polymere, insbesondere von mechanischen Eigenschaften, solche Monomere zuzufügen, deren Brechungsindex nicht ganz so vorteilhaft ist, da selbst dann der Brechungsindex des entstehenden Polymers immer noch hoch genug ist, um für viele Anwendungen geeignet zu sein. Auf diese Weise ermöglichen die erfindungsgemäßen Monomere mehr Flexibilität in der Entwicklung von Polymeren.

Gegenstand der Erfindung sind somit neue Monomere der folgenden Formel PG-Z-VG-(S-Aryl)ₙ, worin n eine ganze Zahl von 2 bis 5 ist, PG eine polymerisierbare Gruppe ist und VG eine Verknüpfungsgruppe ist und Z O, NH oder eine Bindung bedeutet, wobei die polymerisierbare Gruppe mindestens eine polymerisierbare Doppelbindung aufweist und wobei die Verknüpfungsgruppe eine geradkettige, verzweigte oder cyclische Alkylgruppe mit 1 bis 12 Kohlenstoffatomen oder eine Arylgruppe mit 6 bis 18 Kohlenstoffatomen ist, die gegebenenfalls bis zu drei Heteroatome ausgewählt aus O, S und N enthalten kann.

Die erfindungsgemäßen Monomere zeichnen sich dadurch aus, dass sie einerseits eine polymerisierbare Gruppe (PG), die nach der Polymerisation das Gerüst des Polymers bildet, und andererseits mindestens zwei über S gebundene Arylgruppen aufweisen, die den hohen Brechungsindex bewirken. Die polymerisierbare Gruppe und die Arylgruppen sind über eine Verknüpfungsgruppe (VG) miteinander verbunden. Die Verknüpfungsgruppe hat die Aufgabe, die S-ArylGruppen in geeigneter Weise an das Polymergerüst zu binden, damit sich die gewünschten mechanischen und optischen Eigenschaften einstellen. Einerseits soll die Verknüpfungsgruppe den Brechungsindex möglichst wenig beeinträchtigen. Aus diesem Grund werden bevorzugt kurzkettige Alkylgruppen und kleine Arylgruppen in Betracht gezogen. Andererseits darf die Verknüpfungsgruppe nicht zu einer sterischen Behinderung führen, um die mechanischen Eigenschaften nicht zu beeinträchtigen.

Die drei Komponenten werden im Folgenden genauer erläutert. Die polymerisierbare Gruppe kann jede Gruppierung sein, die durch radikalische Polymerisation, insbesondere durch kontrollierte radikalische Polymerisation polymerisierbar ist. Sie liefert das Gerüst des Polymers und trägt daher zu den mechanischen Eigenschaften bei. Bevorzugt werden daher solche Gruppierungen als Gruppe PG eingesetzt, die die mechanischen Eigenschaften verbessern oder zumindest nicht beeinträchtigen. Bevorzugt sind weiterhin solche Gruppierungen, die die optischen Eigenschaften nicht verschlechtern. Die polymerisierbare Gruppe liefert das Gerüst des Polymers und trägt daher zu den mechanischen Eigenschaften bei. Sie kann daher auch abhängig vom Verwendungszweck ausgewählt werden. Alle für die Herstellung von Polymeren, die zu Intraokularlinsen verarbeitet werden, verwendeten polymerisierbaren Gruppen sind hier besonders geeignet. Als Beispiel können Verbindungen genannt werden, die eine endständige Doppelbindung aufweisen, die an einen Arylrest, insbesondere einen Phenyl- oder Naphthylrest, oder an eine kurzkettige Alkyl- oder Alkoxyverbindung gebunden ist. Unter kurzkettigen Verbindungen werden hierbei Verbindungen mit 1 bis 5 Kohlenstoffatomen, insbesondere 1 bis 3 Kohlenstoffatomen verstanden. Besonders bevorzugt werden als polymerisierbare Gruppen Vinyl-, Styryl-, Acrylat- und Methacrylatgruppen verwendet.

Die polymerisierbare Gruppe PG ist an die Verknüpfungsgruppe entweder über ein Sauerstoffatom oder über NR^{c} gebunden, wobei R^{c} H oder eine kurzkettige Alkylgruppe, bevorzugt H oder CH₃ und insbesondere H bedeutet. Die erfindungsgemäß eingesetzten Monomere sind bevorzugt Acryl- und Methacryester bzw. Amide.

Die Verknüpfungsgruppe VG ist eine möglichst kurzkettige Verbindung, an die S-Arylgruppen gebunden sind. Die Verknüpfungsgruppe kann geradkettig oder verzweigt, cyclisch oder aromatisch sein. Bevorzugt werden kurzkettige bzw. cyclische oder aromatische Verbindungen eingesetzt. Als geradkettige oder verzweigte Gruppen kommen insbesondere kurzkettige Alkylgruppen in Betracht, die die S-Arylgruppen an ihren Enden oder auch als Seitenketten tragen können, wobei dann, wenn mehrere S-Arylgruppen an eine kurze Alkylgruppe gebunden sind, die Bindung so erfolgen sollte, dass die Gruppen sich nicht sterisch behindern. Bevorzugt ist daher, dass die S-Arylgruppen endständig gebunden sind. Bevorzugte geradkettige oder verzweigte Alkylgruppen für die Verbindungsgruppe sind daher Isopropyl, tert.-Butyl, Neopentyl und andere tertiäre Alkylgruppen. Bevorzugt werden als Alkylgruppen solche mit 1 bis 8 und insbesondere 1 bis 5 Kohlenstoffatomen eingesetzt.

In einer bevorzugten Ausführungsform ist die Verknüpfungsgruppe VG eine tertiäre Alkylgruppe, an deren Enden S-Arylgruppen gebunden sind, wobei die Bindung an die PG über das tertiäre C-Atom erfolgt. Als cyclische Gruppen sind cyclische Alkylgruppen und auch solche mit Schwefelatomen im Ring zu nennen. Bevorzugt haben die cyclischen Gruppen 5 oder 6 Atome im Ring.

Als Arylgruppe sind insbesondere Phenyl- und Naphthylgruppen zu nennen, wobei Phenylgruppen besonders bevorzugt sind. Alle als Verbindungsgruppen genannten Gruppen können einen oder mehrere Substituenten tragen. Als Substituenten geeignet sind insbesondere Halogene, kurzkettige Alkyl- und Alkoxygruppen sowie Thioalkylgruppen. Besonders bevorzugt als Substituenten sind Chlor, Brom, Iod und Fluor, Methyl, Methoxy und Thiomethyl. Die Anzahl der Substituenten kann 1 bis 5 sein und ist bevorzugt 1 bis 3.

Wesentlicher Bestandteil der erfindungsgemäßen Monomere sind -S-Arylgruppen, die den Brechungsindex in positiver Weise beeinflussen. Die S-Arylgruppen können an beliebigen Stellen der Verbindungsgruppe gebunden sein, solange keine sterische Hinderung oder Beeinträchtigung von Eigenschaften auftritt. Wenn die Verknüpfungsgruppe ein Arylrest ist, können S-Arylgruppen an jedem beliebigen C-Atom, das für eine Bindung zur Verfügung steht, gebunden sein. Bevorzugt sind S-Arylgruppen in meta- und/oder para-Stellung gebunden. Es können auch alle nicht durch die Bindung an die polymerisierbare Gruppe gebundenen Positionen durch S-Arylgruppen besetzt sein.

Weiterhin kann die Verknüpfungsgruppe auch eine Aralkylgruppe sein, wobei hier unter Aralkylgruppe eine Gruppe verstanden wird, die sowohl einen Arylanteil als auch einen oder mehrere Alkylanteile aufweist. Die S-Arylgruppen können an den Arylanteil und/oder den Alkylanteil gebunden sein. Die Verbindung zu der polymerisierbaren Gruppe wird bevorzugt über O bzw. NH hergestellt. Bevorzugt werden Aralkylgruppen verwendet, bei denen die S-Arylgruppen an einen Methylen- oder Ethylenrest gebunden sind, und/oder die Verbindung zu dem polymerisierbaren Anteil über eine Methylen-, Ethylen-, Methoxy- oder Ethoxygruppe erfolgt.

Besonders bevorzugte Kombinationen aus Verknüpfungsgruppen und S-Arylgruppen sind im Folgenden aufgeführt:, wobei X jeweils O oder NR^{c} bedeutet:

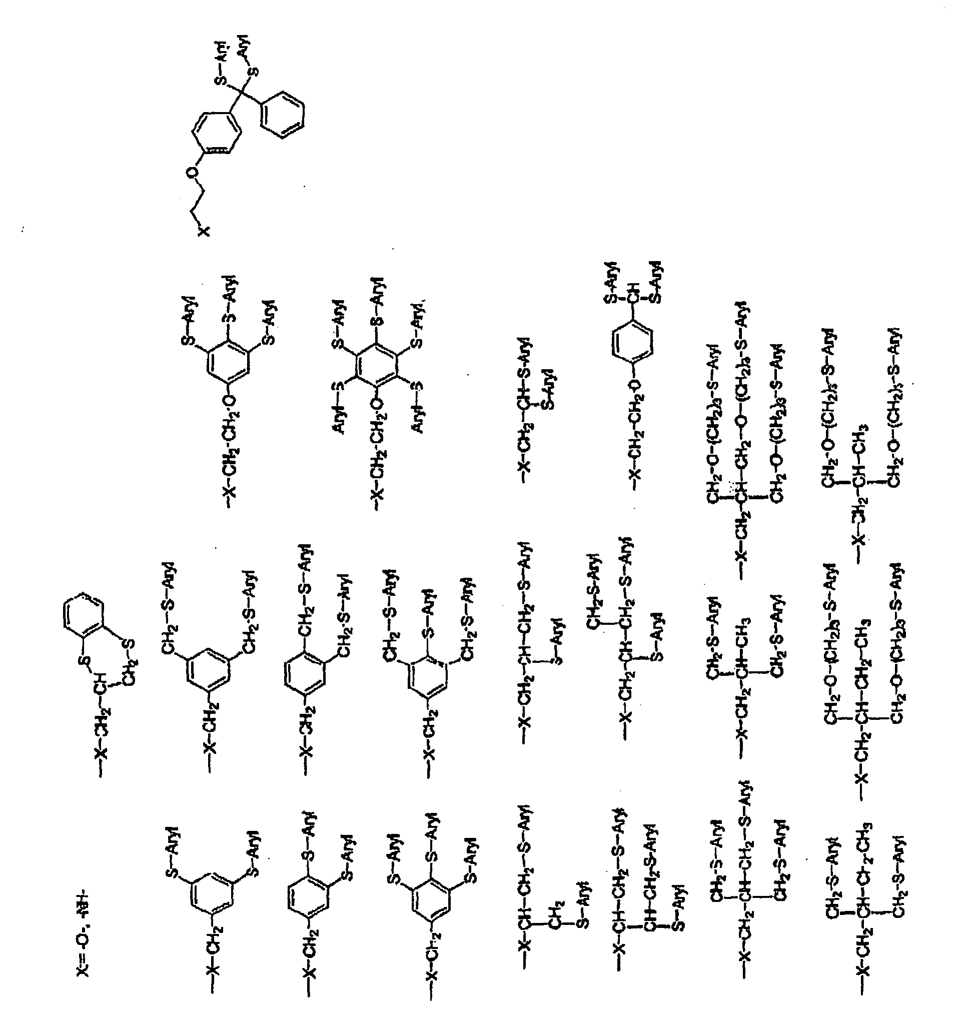

In einer bevorzugten Ausführungsform werden Monomere bereitgestellt mit der Formel (II): 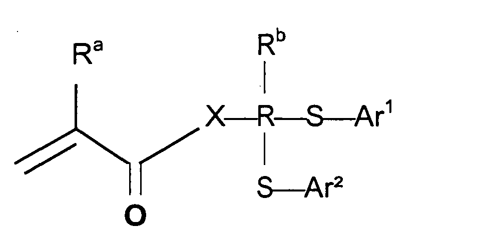 worin X O oder NR^{c} sein kann,
R einen linearen, verzweigten oder cyclischen Kohlenwasserstoffrest mit 1 bis 6 Kohlenstoffatomen bedeutet,
R^{a} Wasserstoff oder einen Methylrest bedeutet,
R^{b} Wasserstoff, einen C₁-C₅-Alkylrest oder S-Ar³ bedeuten kann,
R^{c} Wasserstoff, einen linearen, verzweigten oder cyclischen Kohlenwasserstoffrest mit 1 bis 6 Kohlenstoffatomen oder eine Arylgruppe, bevorzugt Wasserstoff bedeutet,
Ar¹, Ar² und Ar³ jeweils unabhängig voneinander eine Arylgruppe bedeuten, die über eine Bindung oder über (-CH₂)ₙ, wobei n 0, 1, 2 oder 3 sein kann, an S gebunden ist und wobei die Arylgruppe mit 1 bis 4 Substituenten substituert sein kann, ausgewählt aus C₁-C₅-Alkyl, C₁-C₅-Alkoxy, mono- und disubstituiertem Amino, wobei die Substituenten ausgewählt sein können aus Resten R^{c}, wie oben definiert.

Bevorzugt werden als Monomer solche Verbindungen eingesetzt, in denen Ar jeweils einen Phenylrest bedeutet, der 0, 1 oder 2 Substituenten aufweist ausgewählt aus Alkyl- und Alkoxyresten, insbesondere Methyl- und Methoxyresten.

Besonders bevorzugt werden als Monomere die folgenden Verbindungen eingesetzt:

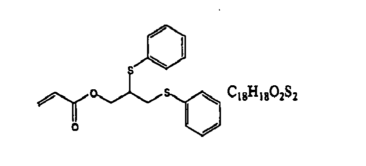

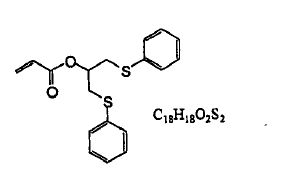

Es wurde überraschenderweise festgestellt, dass aus den erfindungsgemäßen Monomeren hergestellte Polymere besonders gute optische und mechanische Eigenschaften besitzen. Insbesondere der hohe Brechungsindex dieses Materials ist hervorzuheben, der dafür sorgt, dass mit den Monomeren eine Vielzahl von Produkten hergestellt werden können, die für optische Zwecke eingesetzt werden können. In Betracht kommen hier vor allem ophtalmische Produkte, wie Implantate, und jede Art von optischen Linsen, aber auch transparente Überzüge. Mit den erfindungsgemäßen Monomeren hergestellte Polymere haben einen sehr hohen Brechungsindex, der abhängig vom Anteil der erfindungsgemäßen Monomeren weit über 1,6 liegen kann. Da die Wirkung einer optischen Linse bei vorgegebener Geometrie um so stärker ist, je mehr sich der Brechungsindex von dem des umgebenden Mediums unterscheidet und der Brechungsindex von Wasser, der Umgebung einer Intraokularlinse, 1,334 ist, lässt sich die Dicke einer Linse beträchtlich vermindern, wenn der Brechungsindex statt 1,56 weit über 1,6 beträgt. Aus den erfindungsgemäßen Monomeren lässt sich daher ein Polymer herstellen, das einen so hohen Brechungsindex hat, dass Augenimplantate mit sehr dünnem Querschnitt hergestellt werden können, die die optischen Anforderungen erfüllen. Weiterhin sind auch die mechanischen Eigenschaften der aus erfindungsgemäßen Monomeren hergestellten Polymere ausgezeichnet und können außerdem durch Copolymerisation mit anderen Monomeren, die gewünschte Eigenschaften liefern, weiter verbessert werden. Für die Copolymerisation bleibt, wie oben erwähnt, ein breiterer Spielraum, als bei erfindungsgemäßen Polymeren, aufgrund des weitaus höheren Brechungsindex. Darüber hinaus wurde gefunden, dass die Polymere im UV-Bereich intrinsisch sind, d.h. im UV-Bereich ohne zusätzlichen UV-Absorber absorbieren. Außerdem können die Polymere so ausgebildet werden, dass sie sich bei Raumtemperatur falten bzw. rollen lassen und aufgrund der Rückstellkräfte im Auge selbständig entfalten. Bevorzugt werden hierzu hydrophobe Polymere verwendet, deren Wassergehalt unter 4 Gew.-% liegt. Die Glasübergangstemperatur der Polymere wird dabei so eingestellt, dass sie bevorzugt unter Raumtemperatur und typischerweise unter 15°C liegt.

Die erfindungsgemäßen Polymere eigenen sich auf für andere ophtalmische Vorrichtungen, wie Kontaktlinsen, Keratoprothesen, Hornhautringe oder -inlays. Die jeweils optimalen Eigenschaften lassen sich durch Kombination der Monomere gut einstellen.

Aus den erfindungsgemäßen Monomeren können Homopolymere oder Copolymere hergestellt werden. Die Homopolymere haben einen sehr hohen Brechungsindex und sind daher vorteilhaft. Durch Verwendung von Comonomeren können auch andere Eigenschaften optimiert werden. So ist es beispielsweise möglich, die thermische Expansion der Polymeren einzustellen über den Anteil des Vernetzers. Für solche Polymere, die z.B. als Linsen in optischen Geräten eingesetzt werden, die für jede Verwendung sterilisiert werden müssen, ist die thermische Belastbarkeit ein wichtiges Merkmal. Eine Einstellung der thermischen Expansion führt daher zu Materialien, die über längere Zeit ohne nachteilige Wirkung verwendet werden können. Eine weitere wichtige Eigenschaft ist die Klebrigkeit von Polymeren. Für viele Anwendungen ist es notwendig, klebrige Oberflächen zu vermeiden. In solchen Fällen, können mit den erfindungsgemäßen Monomeren fluorhaltige Monomere copolymerisiert werden. Als Comonomere eignen sich dabei Monomere, die den erfindungsgemäß eingesetzten Monomeren in der Struktur gleichen oder ähnlich sind und ein oder mehrere Fluoratome oder CF₃-Gruppen tragen.

Die erfindungsgemäßen Polymere können in Form von statistischen Polymeren oder als Blockcopolymere vorliegen. Die Herstellung der beiden Arten von Polymeren sind dem Fachmann an sich bekannt und bedürfen keiner näheren Erläuterung. Blockcopolymere, die Blöcke mit hohem Brechungsindex verbunden durch andere Blöcke mit geringerem Brechungsindex aufweisen, können Produkte mit sehr interessanten Eigenschaften liefern. Aber auch statistische Copolymere können sehr interessante Eigenschaften aufweisen, da die hoch brechenden Monomere mit Monomeren mit anderen wünschenswerten Eigenschaften in einem breiten Bereich gemischt werden können, was eine hohe Flexibilität und Variabilität ermöglicht.

Die aus den erfindungsgemäßen Monomeren hergestellten Polymeren - sowohl in Form der Homopolymere als auch in Form der Copolymere - sind auch geeignet zum Beschichten von Oberflächen. In einer bevorzugten Ausführungsform kann ein erfindungsgemäßes Polymer zum Überziehen von Metallen verwendet werden, z.B. metallische Oberfläche bei Implantaten, um die Oberfläche inert und hydrophob zu machen.

Die erfindungsgemäßen Monomere können mit an sich bekannten Verfahren oder dazu analogen Verfahren hergestellt werden. Ein Beispiel zur Herstellung der erfindungsgemäßen Verbindungen ist die Reaktion von geeigneten thiolgruppenhaltigen Verbindungen mit Halogenalkoholen. Schwefelhaltige Alkohole bzw. Thiole erhält man mit dem Fachmann auf diesem Gebiet bekannten Verfahren. Ein Beispiel hierzu ist die Umsetzung von Halogenalkoholen oder halogenhaltigen Epoxiden oder die Umsetzung von Epoxiden mit Thiolen. Analog erhält man schwefelhaltige Amine mit dem Fachmann bekannten Verfahren.

In einer weiteren Variante können die erfindungsgemäßen Monomere durch Reaktion von Acrylsäurehalogeniden oder Methacrylsäurehalogeniden mit schwefelhaltigen Alkoholen hergestellt werden.

Die erfindungsgemäßen Monomere können in an sich bekannter Weise mit Polymerisationsverfahren polymerisiert werden. Geeignet sind, ohne darauf beschränkt zu sein, radikalische Polymerisationsverfahren in Form von Substanz-("Bulk-"), Lösungs-, Heterophasen-, Emulsions- oder auch Fällungspolymerisation. Die Initiierung der Polymerisation kann in an sich bekannter Weise erfolgen. Die bekannten und verfügbaren Verfahren sind hier geeignet. Es können dies photochemische Verfahren, thermische Verfahren, durch Redoxsysteme oder auch energiereiche Strahlung initiierte Reaktionen sein. Weiterhin können die Monomere auch durch kontrollierte radikalische Polymerisation, beispielsweise Atomtransferpolymerisation (ATRP), "Reversible Addition Fragmentation Chain Transfer"-(RAFT)-Polymerisation, Nitroxyl mediated polymerization (NMP), Madix usw. polymerisiert werden. Dabei kann die Polymerisation sowohl in Substanz, als auch in Lösung, Emulsion oder durch Fällung erfolgen.

Die erfindungsgemäßen Monomere können entweder zu Homopolymeren oder Copolymeren umgesetzt werden. Copolymere können dabei entweder durch Verwendung verschiedener erfindungsgemäßer Monomere oder aber auch durch Verwendung von einem oder mehreren der erfindungsgemäßen Monomere mit weiteren Monomeren, die andere Eigenschaften beitragen, erfolgen. Als weitere Monomere können die für optische Materialien üblicherweise verwendeten Monomere zugefügt werden. Insbesondere können solche Monomere erwähnt werden, wie sie üblicherweise zur Herstellung von Intraokularlinsen eingesetzt werden, z.B. Phenoxyethylacrylat, Phenoxyethylmethacrylat, Hydrooxyethylmethacrylat und andere Phenoxyacrylate und -methacrylate.

In einer bevorzugten Ausführungsform werden die erfindungsgemäßen Monomere durch Photopolymerisation polymerisiert. Als Photoinitiatoren können die für derartige Polymerisationen bekannten Photoinitiatoren eingesetzt werden.

Um die mechanischen Eigenschaften des aus den Monomeren zu polymerisierenden Polymermaterials zu verändern, insbesondere um die Flexibilität zu beeinflussen, wird in einer bevorzugten Ausführungsform ein Vernetzer verwendet. Als vernetzende Monomere werden solche Verbindungen eingesetzt, die mindestens zwei polymerisierbare Gruppen aufweisen. Beispiele für geeignete polymerisierbare Gruppen sind Vinyl-, Acrylat- und Methacrylatgruppen. Geeignet sind gegebenenfalls auch Verbindungen mit zwei reaktiven Gruppen, z.B. Thiolgruppen. Im Stand der Technik sind viele vernetzende Monomere bekannt, die erfindungsgemäß eingesetzt werden können. Beispiele sind Divinylbenzol, Dithioresorcin, Bisphenol-A-methacrylat, Ethylenglycoldimethacrylat; Diethylenglycoldimethacrylat; Allylmethacrylat; 1,3-Propandioldimethacrylat; 2,3-Propandioldimethacrylat; 1,6-Hexandioldimethacrylat; 1,4-Butandioldimethacrylat; CH₂=C(CH₃)C(=O)O-(CH₂CH₂O)ₙ-C(=O)C(CH₃)=CH₂, worin n eine ganze Zahl von 1 bis 3 ist. Besonders gute Eigenschaften werden jedoch erhalten, wenn auch als Vernetzer thioarylgruppenhaltige Verbindungen eingesetzt werden.

Als besonders geeignet haben sich Verbindungen der Formel III 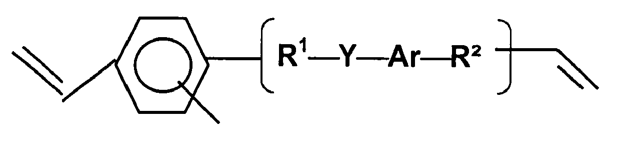 erwiesen, die an beiden Enden endständig ungesättigte Gruppen tragen, wobei Y O oder S, bevorzugt S bedeuten kann,
Ar ein aromatischer, insbesondere Phenylrest ist, der mit 0 oder 1 bis 4 Substituenten substituiert sein kann, die ausgewählt sind aus C₁-C₅-Alkylresten, C₁-C₅-Alkoxyresten und Halogenen, wobei R¹ und R² eine Bindung oder ein (CH₂)ₘ-Rest, worin m 1, 2 oder 3 ist, bedeuten.

Weiterhin besonders geeignet als Vernetzer sind Verbindungen der allgemeinen Formel IV

Y-S-B-S-Y,

worin Y ein vinylgruppenhaltiger Rest, insbesondere ein Styrylmethylrest ist und B ein Aryl- oder geradkettiger oder verzweigter Alkylrest mit 1 bis 5 Kohlenstoffatomen ist. Wenn B ein Arylrest ist, können die beiden S-Y-Gruppen in meta-, ortho- und para-Stellung gebunden sein.

Die erfindungsgemäß als vernetzende Monomere bereitgestellten Verbindungen können in an sich bekannter Weise hergestellt werden. Z.B. erhält man durch Reaktion eines Dithiols mit einem vinylisch ungesättigten Halogenid 4-Vinylbenzylchlorid.

Die erfindungsgemäßen Polymere werden hergestellt unter Verwendung von 50 bis 100% der erfindungsgemäßen Monomere der Formel 1. Weiterhin können 0 bis 49 Gew.-% an weiteren Monomeren copolymerisiert werden. Zusätzlich können bis zu 15 Gew.-%, bevorzugt bis zu 10 Gew.-% an vernetzenden Monomeren zugefügt werden. Der Anteil an Vernetzer liegt bevorzugt in einem solchen Bereich, dass unlösliche oder quellbare Polymerisate erhalten werden.

In einer weiteren Ausführungsform werden die erfindungsgemäßen Monomere zur Herstellung von Pfropfcopolymerisaten auf bereits bekannten Polymeren verwendet.

Die erfindungsgemäß erhaltenen Polymere haben im Vergleich zu bekannten Polymeren einen sehr hohen Brechungsindex, der weit über 1,6 liegt. Wenn der Brechungsindex noch weiter erhöht werden soll, können dem Polymermaterial anorganische Nanopartikel zugefügt werden, wie es beispielsweise in WO 03/001278 A2 beschrieben wird. Besonders bevorzugt wird das Polymermaterial mit Titandioxid in Form von Rutil und/oder Anatas kombiniert.

Erfindungsgemäß werden Monomere bereitgestellt, die Polymere liefern mit besonders vorteilhaften optischen Eigenschaften. Diese Materialien können z.B. für optische Bauelemente und ophthalmologische Implantate verwendet werden. Bevorzugt werden sie zur Herstellung von Intraokularlinsen, künstlicher Hornhaut, optischen Linsen, z.B. für Mikrokameras oder Endoskope, von optischen Datenspeichern, Brillengläsern, hochbrechenden Filme und optischen Fasern verwendet.

Die aus den erfindungsgemäßen Monomeren erhaltenen Polymermaterialien sind aufgrund ihrer Eigenschaften vielfältig einsetzbar, vor allen Dingen im optischen Bereich. Die erfindungsgemäßen Polymere können für ophthalmologische Implantate, z.B. Intraokularlinsen, künstliche Hornhaut usw., optische Linsen für Kameras, z.B. Mikrokameras, Endoskope, optische Datenspeicher, Brillengläser, hochbrechende transparente Filme und Beschichtungen und optische Fasern verwendet werden.

Gegenstand der Erfindung ist daher auch die Verwendung eines Polymermaterials, wie oben definiert, zur Herstellung von optischen Bauelementen und ophthalmologischen Präparaten.

Die Erfindung wird durch die folgenden Beispiele erläutert, die jedoch in keiner Weise als beschränkend anzusehen sind.

Die erhaltenen Produkte wurden mit ¹H- und ¹³C-NMR-Spektroskopie untersucht. Es wurde ein Spektrometer INNOVA 500 (Varian Inc.) verwendet und die Messungen wurden an diesem Spektrometer bei Raumtemperatur (21 °C) mit folgenden Messfrequenzen durchgeführt: ¹H-NMR: 499,84 MHz, ¹³C-NMR: 125,69 MHz. Als Lösungsmittel wurden CD₂CL₂ und CD₃OD verwendet.

### Beispiel 1

### Synthese von

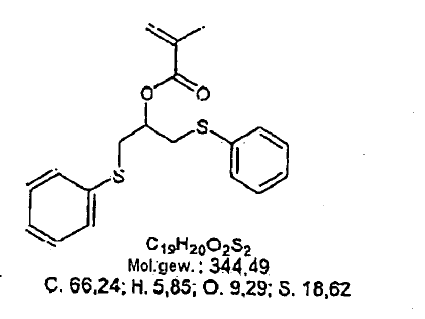

### Reaktionsgleichung

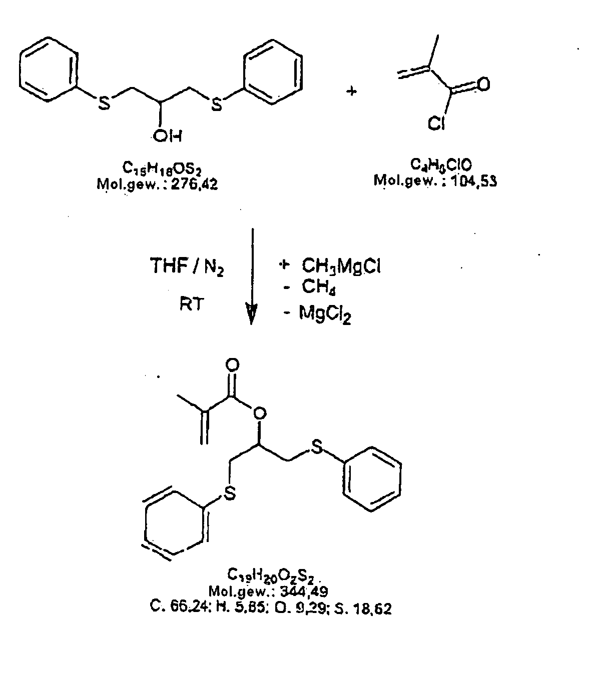

### Reagenzien:

### Methacryloylchlorid

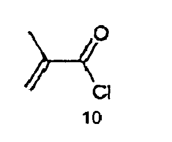

### Methylmagnesiumchlorid

### Experimenteller Teil

### (2-Methacrylsäure-2-phenylsulfanyl-1-phenylsulfanylmethyl-ethylester)

1,3-Bisphenylsulfanyl-propan-2-ol wurde mit Methylmagnesiumchlorid und Methacrylsäurechlorid in äquimolaren Anteilen umgesetzt (Ansatz je 0,03618 Mol).

1,3-Bisphenylsulfanyl-propan-2-ol (MW = 276,42): (10 g = 0,03618 Mol) wurden in THF, das vorher über Na/K destilliert worden war, gelöst. Zu dieser Lösung wurden 0,03618 Mol Methylmagnesiumchlorid [Acros, 22 gew.-%ige Lösung in THF] (MW = 74,79) = (2,71 g (entspricht 12,32 g einer 22 gew.-%igen Lösung)) zugegeben. Diese Lösung wurde dann durch einen Tropftrichter zu folgender Lösung langsam zugegeben: 0,03618 Mol Methacryloylchlorid [97,0% (GC)] (MW = 104,53) = (3,78 g (d.h. 3,89 g des 97,0%igen Präparats)) in ca. 100 ml THF gelöst. Die Reaktionslösung erwärmte sich nicht nennenswert, eine Kühlung war nicht nötig; die Lösung blieb klar, leicht gelblich. Bei Raumtemperatur wurde unter Stickstoffatmosphäre gerührt. Die Reaktion wurde über DC kontrolliert. Anschließend wurde ca. 2 h lang auf 45 bis 50°C erwärmt. Es wurde weiter über Nacht bei Raumtemperatur unter N₂-Atmosphäre gerührt. Die Filtration der Reaktionslösung erfolgte über eine mit Aluminiumoxid [Acros, Aluminiumoxid, activated, basic] und Seesand beschichtete G3-Fritte, die vorher mit THF aufgeschlämmt wurde. Anschließend wurde das THF am Rotationsverdampfer entfernt und das Produkt dann mit Säulenchromatographie weiter gereinigt.

### Beispiel 2

### Synthese von

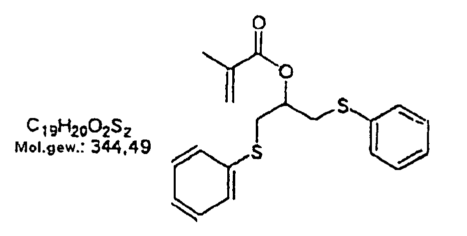

### Reaktionsgleichung

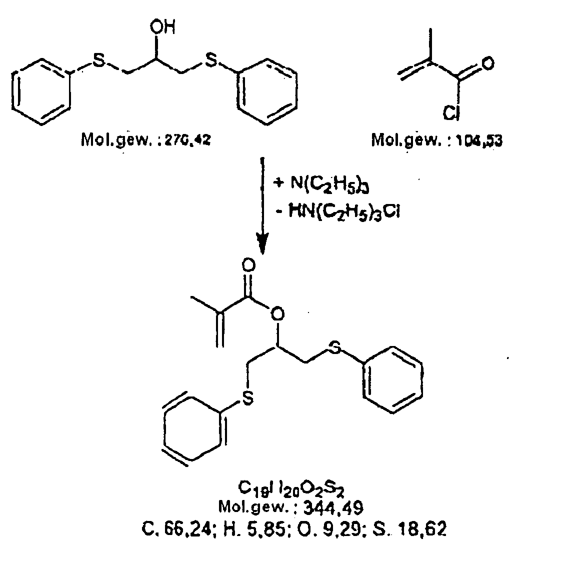

### Chemikalien

### Methacryloylchlorid (wie oben definiert)

### Experimenteller Teil

1,3-Bisphenylsulfanyl-propan-2-ol wurde mit Methacryloylchlorid umgesetzt. In einen vorher ausgeheizten 250-ml-Dreihalskolben mit Kühler und N₂-Einleitung wurden die folgenden Reagenzien gegeben: 0,03 Mol Methacryloylchlorid (3,88 g, 97%ig), ca. 60 ml inhibitorfreies vorher destilliertes THF als Lösemittel. in einen Tropftrichter wurden 8,29 g 1,3-Bisphenylsulfanyl-propan-2-ol (0,030 Mol) und 2,94 g Triethylamin (0,030 Mol) (99 gew.-%ig) zugefügt. Die Lösung aus dem Tropftrichter wurde langsam in den Dreihalskolben zutropfen gelassen. Da die Reaktion nicht exotherm war, war keine Kühlung notwendig. Es bildete sich ein weißer Niederschlag aus (NEt₃)HCl. Bei Raumtemperatur wurde 1,5 Stunden lang weiter gerührt und der entstehende Niederschlag, ca. 2,8 g (NEt₃)HCl dann abfiltriert und anschließend das THF am Rotationsverdampfer entfernt. Man erhielt eine gelbe zähe Flüssigkeit. Die Rohausbeute war 14 g. Zur Reinigung des erhaltenen Produktes wurde die erhaltene Flüssigkeit (14 g) in ca. 50 ml CH₂Cl₂ gelöst und dann mit 5%iger NaHCO₃-Lösung ausgeschüttelt. Es bildete sich eine Emulsion, die durch Zugabe von NaCl gebrochen wurde. Anschließend wurde über Na₂SO₄ getrocknet und dann am Rotationsverdampfer und anschließend mit einer Hybridölpumpe eingeengt. Man erhielt eine gelbe viskose Flüssigkeit in einer Ausbeute von ca. 9 g, was 0,0267 Mol oder 89% der Theorie entsprach. Das Produkt wurde anschließend noch chromatographisch aufgereinigt.
¹H-NMR für C₁₉H₂₀O₂S₂ (Molekulargewicht 344,49) in CD₂Cl₂
¹H-NMR: 499,84 MHz in CD₂Cl₂
7,36-7,34 ppm (4 H, m, H3), 7,27-7,24 ppm (4 H, m, H2), 7,19-7,17 ppm (2 H, m, H1), 5,90 ppm (1H, m, H9), 5,48 ppm (1 H, m, H9'), 5,12 ppm (1 H, m, H6), 3,29 ppm (4 H, m, H5), 1,80 ppm (3 H, m, H8)
¹³C-NMR: 125,69 MHz in CD₂Cl₂
166,66 ppm (C7), 136,22 ppm (C4), 135,73 ppm (C8), 129,89 ppm (C3), 129,28 ppm (C2), 126,70 ppm (C1), 126,09 ppm (C9), 72,17 ppm (C6), 36,53 ppm (C5), 18,16 ppm (C10)

### Beispiel 3

### Synthese von

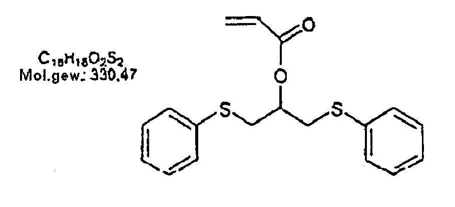

### Reaktionsgleichung

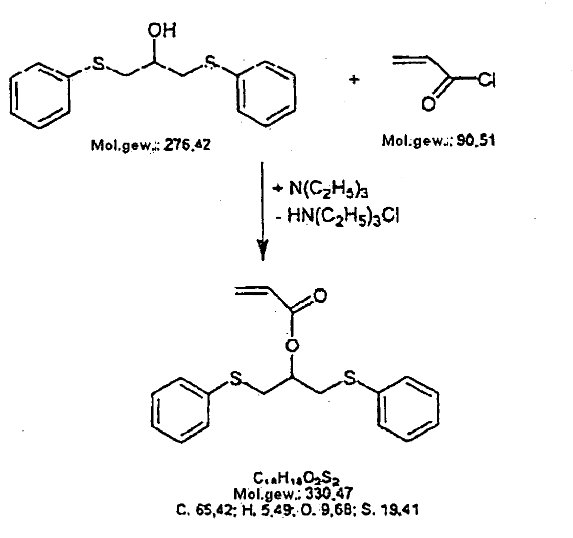

### Reagenzien

### Acryloylchlorid

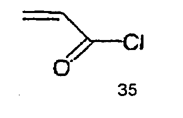

### Experimenteller Teil

In einen vorher ausgeheizten Dreihalskolben mit Kühler und N₂-Einleitung wurden 0,03 Mol Acryloylchlorid, wie vorher definiert, (2,828 g, 96 gew.-%ig) und 50 bis 100 ml inhibitorfreies THF (vorher dest.) als Lösungsmittel gegeben. In einen Tropftrichter wurden 8,29 g 1,3-Bisphenylsulfanylpropan-2-ol = 0,030 Mol und 0,030 Mol Triethylamin [99%], (4,1 ml) gegeben. Aus dem Tropftrichter wurde die Lösung langsam zutropfen gelassen. Da die Reaktion nicht exotherm ist, ist keine Kühlung notwendig. Es bildete sich ein weißer Niederschlag aus (NEt₃)HCl. Es wurde weitere 1,5 h bei Raumtemperatur gerührt und anschließend der Niederschlag abfiltriert und das THF am Rotationsverdampfer entfernt. Man erhielt eine gelbe zähe Flüssigkeit in einer Ausbeute von ca. 9,5 g. Zur Reinigung wurde das Produkt in 50 ml inhibitorfreiem THF gelöst und über eine Alox-Fritte filtriert, um mögliche Inhibitorreste zu entfernen. Das THF wurde am Rotationsdampfer entfernt und anschließend mit einer Hybridpumpe (1,5 bis 1,5 mbar) restliches THF entfernt. Man erhielt eine gelbe viskose Flüssigkeit in einer Ausbeute von ca. 6,8 g, was 0,02067 Mol oder 68,9% der Theorie entspricht.
¹HNMR für C₁₉H₂₀O₂S₂ (Molekulargewicht 344,49) in CD₂Cl₂ 7,33-7,37 ppm (4 H, m, H3), 7,24-7,29 ppm (4 H, m, H2), 7,16-7,21 ppm (2 H, m, H1) 6,225 ppm (1 H, dd, ²J(7',6) = 17,1 Hz, ²J(7', 7) = 1,22 Hz, H7' (trans)) 5,93 ppm (1 H, dd, ²J(6,7) = 10,5 Hz, ²J(6,7') = 17,3 Hz, H6) 5,755 ppm (1 H, dd, ²J(7,6) = 10,4 Hz, ²J(7,7') = 1,22 Hz, H7(cis)) 5,13-5,15 ppm (1 H, m, H5)
3,27 ppm (4 H, m, H4)

### Beispiel 4

### Synthese von

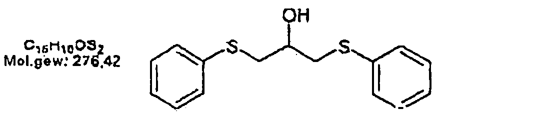

### Reaktionsgleichung

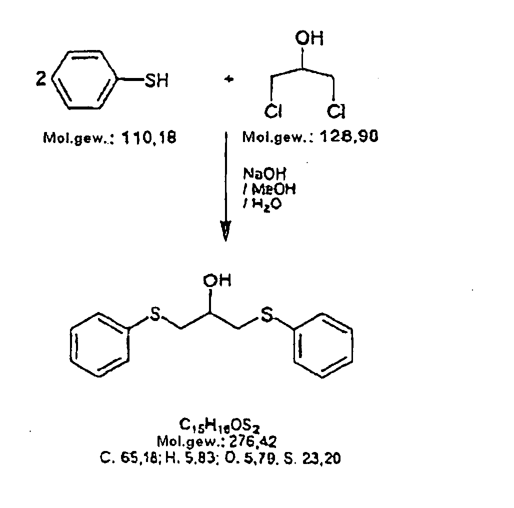

### Reagenzien

### Thiophenol [>98%]

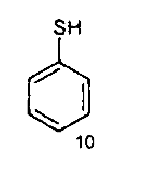

### 1,3-Dichlor-2-propanol [99%]

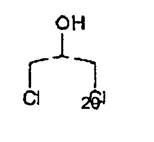

### Natriumhydroxid, Pellets mit 99,998%

### Experimenteller Teil

In einen 500-ml-Dreihalskolben mit Kühler, Tropftrichter und N₂-Einleitung wurden unter N₂-Atmosphäre zuerst gegeben: 0,05 Mol 1,3-Dichlor-2-propanol [99%] (6,449 g) gelöst in 100 ml Methanol. In einen Tropftrichter wurden gegeben: 0,1 Mol Thiophenol [98%], (11,018 g) in 50 ml destilliertem Wasser gelöst, zusammen mit 0,1 Mol NaOH; die Mischung wurde langsam der 1,3-Dichlor-2-propanol-Lösung zugetropft. Nach dem 2/3 der Mischung zugegeben worden waren, entstand ein weißer Niederschlag mit leicht rosa Verfärbung und die Reaktionslösung erwärmte sich. Weitere 50 ml Methanol wurden zugegeben und es wurde über Nacht am Rückfluss (70°C) erhitzt. Der Verlauf der Reaktion wurde über DC verfolgt, wobei CH₂Cl₂ als Laufmittel und Thiophenol als Vergleich verwendet wurden. Am nächsten Tag war die Reaktionslösung klar, leicht gräulich und mit öligen Perlen versetzt. Die Reaktionszeit betrug ca. 20 Stunden bei 70°C.

Die Lösung wurde zuerst am Rotationsverdampfer eingeengt und dann mit CH₂Cl₂ ausgeschüttelt (dreimal mit ca. 100 ml) und über Na₂SO₄ getrocknet.

Dann wurde zuerst am Rotationsverdampfer und dann an der Hybridölpumpe (p = 1,8-1,6 mbar, T = 30°C) eingeengt.

### Charakterisierung:

1,3-Bisphenylsulfanyl-propan-2-ol: 13,63 g einer gelblichen öligen Flüssigkeit (0,0481 Mol); Ausbeute 98,6% der Theorie bezogen auf 0,05 Mol 1,3-Dichlor-2-propanol;
¹H-NMR: 7,32-7,35 ppm (4 H, m, H3), 7,24-7,29 ppm (4 H, m, H2), 7,17-7,21 ppm (2 H, m, H1), 3,78-3,82 ppm (1 H, m, ²J(5,6) = 3,7 Hz, H6), 3,18-3,22 ppm (2H, dd, ²J(4,4') = 13,9 Hz, ²J(4,5) = 4,9 Hz, H5), 3,01-3,05 ppm (2 H, dd, ²J(4',4) = 13,7 Hz, ²J(4',5) = 7,3 Hz, H5'), 2,71 ppm (1 H, d, ²J(6,5) = 3,4 Hz, -OH)
¹³C-NMR: 125,69 MHz in CD₃OD
137,51 ppm (C4), 130,50 ppm (C3), 130,02 ppm (C2), 127,17 ppm (C1), 70,08 ppm (C6), 40,45 ppm (C5)

### Beispiel 5

### Synthese von

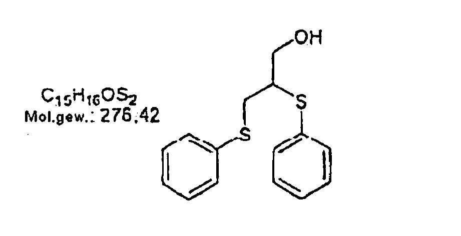

### Reaktionsgleichung

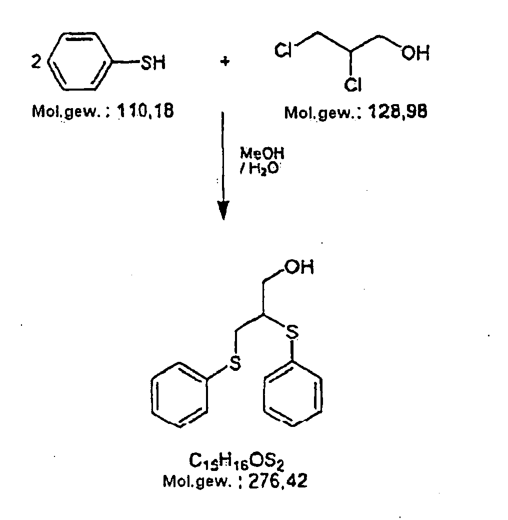

### Reagenzien

### Thiophenol (wie in Beispiel 4)

### 2,3-Dichlor-1-propanol [97,0% (GC)]

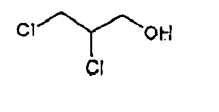

### Natriumhydroxid, Pellets 99,998%

### Experimenteller Teil

In einen 500-ml-Dreihalskolben, der mit Kühler, Tropftrichter, N₂-Einleitung ausgestattet war, wurden unter N₂-Atmosphäre 0,05 Mol 2,3-Dichlor-1-propanol [97% (GC) (6,449 g)] gelöst in 100 ml Methanol gegeben. In einen Tropftrichter wurden 0,1 Mol Thiophenol [98%] (11,018 g) in 50 ml destilliertem Wasser gelöst und 0,1 Mol NaOH, (4,0 g) gelöst in 50 ml destilliertem Wasser, zugegeben. Diese Lösung wurde langsam der 1,3-Dichlor-2-propanol-Lösung zugetropft. Dabei entstand eine leicht rosa Verfärbung und die Reaktionslösung erwärmte sich. Der Verlauf der Reaktion wurde über DC verfolgt, wobei CH₂Cl₂ als Laufmittel und Thiophenol als Vergleich verwendet wurden. Weitere 50 ml Methanol wurden zugegeben und es wurde über Nacht am Rückfluss (70°C) erhitzt. Am nächsten Tag war die Reaktionslösung klar, leicht gräulich und mit öligen Perlen versetzt. Die Reaktionszeit war bei 70°C ca. 20 h.

Die Lösung wurde zuerst am Rotationsverdampfer eingeengt und dann mit CH₂Cl₂ ausgeschüttelt (dreimal mit ca. 100 ml) und über Na₂SO₄ getrocknet. Dann wurde zuerst an einem Rotationsverdampfer und dann an der Hybridölpumpe eingeengt.

Es wurden 12,85 g (0,0465 Mol) einer gelblichen öligen Flüssigkeit erhalten. Dies entsprach einer Ausbeute von 93% der Theorie bezogen auf 0,05 Mol 2,3-Dichlor-1-propanol.

### Charakterisierung:

Das Produkt wurde mit ¹H-NMR + ¹³C-NMR in CD₃-OD untersucht: Es lag ein Isomerengemisch vor mit ca. 80% der Verbindung zu 20% des 2-Propanolderivats.

Der Brechungsindex wurde bestimmt mit: η = 1,6255/T = 25,3°C

### Beispiel 6

### Synthese von

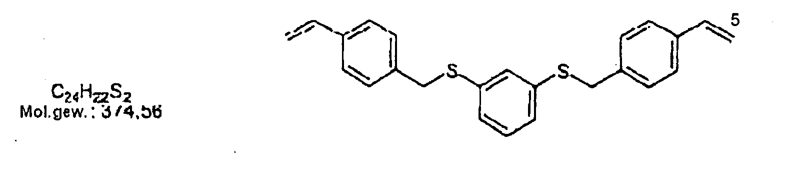

### Reaktionsgleichung

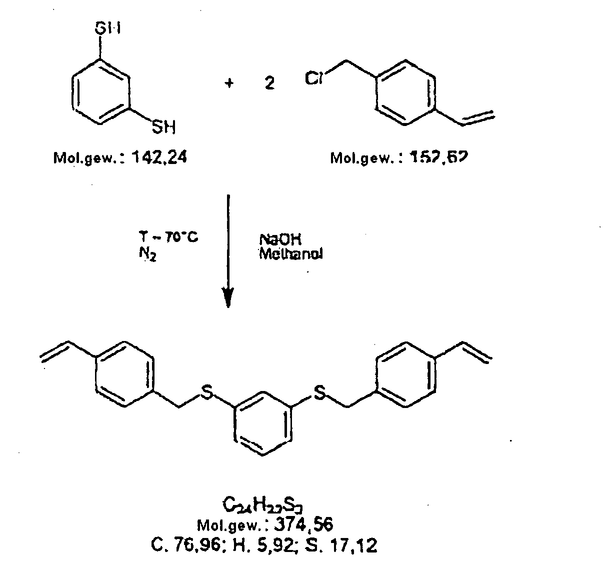

### Reagenzien

### 1,3-Benzoldithiol, 99%

### 4-Vinylbenzylchlorid, technisch 90% (GC)

### Experimenteller Teil

In einen 250-ml-Dreihalskolben, der mit Kühler, Tropftrichter und N₂-Einleitung ausgestattet war, wurden unter Rühren und N₂-Atmosphäre nacheinander 0,01 Mol 1,3-Dithiobenzol [99%] (1,437 g) und 0,02 Mol NaOH (ca. 0,8 g) gelöst in ca. 30 ml destilliertem Wasser gegeben.

Es entstand ein farbloser kristalliner Niederschlag. Der Ansatz wurde auf 40°C erwärmt und 80 ml Methanol wurden zugegeben. Dabei entstand eine milchige Lösung. Anschließend wurden 0,02 Mol 4-Vinylbenzylchlorid [≥90%] (3,0524 g) zugegeben, wobei sich eine Emulsion bildete. Um den restlichen Anteil des 4-Vinylbenzylchlorids zu lösen, wurden weitere 60 ml Methanol zugegeben. Der Ansatz wurde auf 70°C erwärmt und dann über Nacht am Rückfluss gehalten (ca. 65°C). Nach 23 h Reaktionszeit wurde die Reaktionslösung abfiltriert und mit Ether (dreimal mit je 80 ml) extrahiert. Die vereinigten Etherphasen wurden dann zuerst mit 80 ml 1 n NaOH und dann mit destilliertem Wasser ausgeschüttelt und dann über Natriumsulfat getrocknet und am Rotationsverdampfer eingeengt (35°C, p = 700-750 mbar).

Es wurden 1,82 g einer gelblichen öligen Flüssigkeit erhalten. Methanol wurde zugegeben und es wurde auf 66°C erhitzt. Dabei löste sich fast alles und die gelbe Farbe verschwand. Anschließend wurde heiß filtriert und der Filter leicht erwärmt. Dabei fielen sofort farblose Nadeln aus. Es wurde über eine G3-Fritte abgesaugt und dann an einer Hybridölpumpe getrocknet (erst p = 120 mbar, dann 0,12 mbar). Die Ausbeute war 0,76 g (0,00203 Mol), was 20,3% der Theorie entsprach.
¹H-NMR: 499,84 MHz in CD₂Cl₂
7,33 ppm (4H, d, ²J(4,5) = 8,1 Hz, H4), 7,22 ppm (4 H, d, ²J(5,4) = 8,1 Hz, H5), 7,185-7,20 ppm (1 H, m, H11), 7,01-7,16 ppm (3 H, m, 2 H9, 1 H10), 6,68 ppm (2 H, q, ²J(2,1') = 11,0 Hz, ²J(2,1) = 17,6 Hz, H2), 5,73 ppm (2 H, dd, ²(1,1') = 1,0 Hz, ²J(1,2) = 17,6 Hz, H1), 5,22 ppm (2 H, dd, ²J(1',2) = 11,0 Hz, ²J(1',1) = 1,0 Hz, H1'), 4,05 ppm (4 H, s, H7),
¹³C-NMR: 125,69 MHz in CD₂Cl₂
C3 137,42 ppm, C6 137,25 ppm, C8 136,87 ppm, C2 136,58 ppm, C11 130,23 ppm, C10 129,33 ppm, C5 129,26 ppm, C9 127,59 ppm, C4 126,53 ppm, C1 113,93 ppm, C7 38,56 ppm

### Beispiel 7

1,0 g des Monomers von Beispiel 1 wurden mit 0,2% Irgarcure 2022 gemischt und zwischen zwei silanisierte Objektträger, die durch einen Spacer mit 0,9 mm Dicke getrennt waren, gefüllt. Die Photopolymerisation wurde unter N₂-Atmosphäre mit einer UV-Lampe der Marke "Super Actinic" (Lampe TL-D15W/03, λₘₐₓ = 420 nm, Bestrahlungsabstand 18 cm) 2 Stunden lang durchgeführt. Die Vernetzung der Polymere wurde durch Zugabe von 3% Ethylenglycoldimethacrylat erreicht.

## Patentansprüche

1. Monomer der Formel PG-Z-VG-(S-Aryl)ₙ, worin n eine ganze Zahl von 2 bis 5 ist, PG eine polymerisierbare Gruppe ist und VG eine Verknüpfungsgruppe ist und Z O, NH oder eine Bindung bedeutet, wobei die polymerisierbare Gruppe mindestens eine polymerisierbare Doppelbindung aufweist und wobei die Verknüpfungsgruppe eine geradkettige, verzweigte oder cyclische Alkylgruppe mit 1 bis 12 Kohlenstoffatomen oder eine Arylgruppe mit 6 bis 18 Kohlenstoffatomen ist, die gegebenenfalls bis zu drei Heteroatome ausgewählt aus O, S und N enthalten kann.

2. Monomer nach Anspruch 1, **dadurch gekennzeichnet, dass** PG ein Acrylat- oder Methacrylatrest ist.

3. Monomer nach Anspruch 1, **dadurch gekennzeichnet, dass** PG ein Alkylstyryl oder Acetonylrest ist.

4. Monomer nach einem der vorhergehenden Ansprüche, **dadurch gekennzeichnet, dass** n 2 oder 3 ist.

5. Monomer nach einem der vorhergehenden Ansprüche, **dadurch gekennzeichnet, dass** Z O bedeutet.

6. Monomer nach einem der vorhergehenden Ansprüche, **dadurch gekennzeichnet, dass** VG ein unsubstituierter oder substituierter Phenyl- oder Naphthylrest ist, wobei gegebenenfalls die Substituenten ausgewählt sind aus Halogen, Alkylresten mit 1 bis 3 C-Atomen, Alkoxyresten mit 1 bis 3 C-Atomen oder Thioalkoxygruppen mit 1 bis 3 C-Atomen.

7. Monomer nach einem der vorhergehenden Ansprüche, **dadurch gekennzeichnet, dass** VG eine Phenyl- oder Naphthylgruppe ist, die gegebenenfalls 1 bis 3 Substituenten ausgewählt aus Chlor, Brom, Jod, -CH₃, -OCH₃ und -SCH₃ aufweist.

8. Monomer nach einem der vorhergehenden Ansprüche, **dadurch gekennzeichnet, dass** Z O-CH₂- bedeutet.

9. Monomer nach einem der vorhergehenden Ansprüche, **dadurch gekennzeichnet, dass** VG eine lineare oder verzweigte Alkylgruppe mit 1 bis 5 Kohlenstoffatomen und 1 bis 3 Sauerstoffatomen bedeutet.

10. Monomer nach einem der vorhergehenden Ansprüche, **dadurch gekennzeichnet, dass** VG ein Rest ist ausgewählt aus n-Propyl, Isopropyl, n-Butyl, Isobutyl, n-Pentyl, Isopentyl, Neopentyl oder lsohexyl oder tertiären Alkylresten.

11. Monomer nach einem der vorhergehenden Ansprüche, **dadurch gekennzeichnet, dass** VG ein geradkettiger oder verzweigter Alkylrest ist, an dessen endständigen C-Atomen jeweils S-Arylgruppen gebunden sind.

12. Monomer nach einem der vorhergehenden Ansprüche, **dadurch gekennzeichnet, dass** VG eine Arylalkylgruppe ist, bei der der Arylanteil ausgewählt ist aus Phenyl und Naphthyl und der Alkylanteil ausgewählt ist aus C₁-C₄ Alkyl.

13. Polymerisat, erhalten aus einem oder mehreren Monomeren, wie in Anspruch 1 definiert.

14. Polymerisat nach Anspruch 13 erhalten aus mindestens 50 % Monomeren der Formel 1, wie in einem der Ansprüche 1 bis 12 definiert.

15. Polymerisat nach Anspruch 13 oder Anspruch 14, **dadurch gekennzeichnet, dass** es ein Co-, Ter- oder Tetrapolymerisat ist, bei dem mindestens 50 % der Monomere Monomere der Formel I, wie in einem der Ansprüche 1 bis 12 definiert, sind.

16. Polymerisat nach einem der Ansprüche 13 bis 15, **dadurch gekennzeichnet, dass** 0 bis 50 % eines Comonomers ausgewählt aus Styrol, POEMA, POEA und/oder HEMA enthalten sind.

17. Polymerzusammensetzung erhalten durch Polymerisation von 50 bis 99 % eines oder mehrerer Monomere der Formel I, 0 bis 49 % eines vernetzenden Monomers, das zwei polymerisierbare Gruppen aufweist und 0 bis 49 % weiteren Monomeren.

18. Polymerzusammensetzung, **dadurch gekennzeichnet, dass** als Vernetzer eine Styrol-, Acrylat- und/oder Methacrylatverbindung mit mindestens zwei polymerisierbaren Gruppen vorhanden ist.

19. Polymerzusammensetzung nach einem der vorhergehenden Ansprüche, **dadurch gekennzeichnet, dass** als Vernetzer eine Verbindung der Formel Y-S-A-S-Y, wobei A eine lineare oder verzweigte Alkylkette mit 1 bis 8 Kohlenstoffatomen oder eine Arylgruppe mit 6 bis 18 Kohlenstoffatomen ist und Y eine Alkylstyrylgruppe ist, deren Alkylrest 1 bis 3 Kohlenstoffatome aufweisen kann.

20. Verwendung eines Monomers nach einem der Ansprüche 1 bis 12 zur Herstellung von ophthalmischen Implantaten oder optischen Systemen.
